# EUROPEAN PATENT APPLICATION

(11) **EP 2 172 193 A1**
(43) Date of publication of application: **07.04.2010**
(21) Application number: 08165747.0
(22) Date of filing: 02.10.2008
(51) Int. Cl.: A61K 9/50, A61K 9/51, A61K 31/00

(54) **Improved nanoparticulate compositions of poorly soluble compounds**

(71) Applicant: Capsulution Nanoscience AG, 12489 Berlin (DE)
(72) Inventor: The designation of the inventor has not yet been filed
(74) Representative: Kilger, Ute

(57) **Abstract**

The present invention relates to a method for the production of a nanoparticulate pharmaceutical composition. The method comprises the steps of a) suspending in water a poorly soluble active ingredient without the presence of a detergent, b) mechanically treating said suspension to obtain particles comprising the active ingredient with an effective average size of less than about 5000 nm, c) contacting said active ingredient or suspension with a first polyelectrolyte during and/or before mechanically treating, d) optionally contacting said suspension with a one or more second or further polyelectrolytes during, before and/or after mechanically treating, e) optionally drying said suspension. The invention also pertains to the pharmaceutical compositions obtained by the method of the invention.

## Description

### Technical field of the invention

The present invention is in the field of drug formulation. In particular the present invention relates to methods of making improved nanoparticulate compositions of poorly soluble compounds and nanoparticulate pharmaceutical compositions.

### Background of the invention

The application of drugs with a poor solubility in water is a general problem in pharmaceutics. Poor solubility, *inter alia,* leads to poor bioavailability of these drugs. In general, bioavailability of active ingredients can be improved by increasing particle surfaces, e.g. by the provision of small particles of these active ingredients (i.e. micro- or nanoparticulate pharmaceutical compositions). Another problem is the stabilization of pharmaceutical compositions. Solubility and stability of pharmaceutical compositions is often mediated by the addition of additives such as surfactants (detergents). Poorly soluble drugs may for example be coated by layers of polyelectrolytes as described in WO2004/030649A2 or WO2007/031345A2. Pharmaceutical compositions comprising surface modifiers that are attached to the surface of poorly soluble drugs and processes for obtaining such Pharmaceutical compositions are for example described in EP0644755B1 I and EP1490025B1.

### Summary of the invention

It was an object of the present invention to provide improved nanoparticulate compositions of poorly soluble compounds avoiding the use of detergents during the process of preparation of these nanoparticulate compositions of poorly soluble compounds. It was also an object of the invention to provide methods of making improved nanoparticulate compositions of poorly soluble compounds. In particular, the present invention provides a method for the production of a nanoparticulate pharmaceutical composition comprising an active ingredient comprising the steps of:
a) suspending in water a poorly soluble active ingredient without the presence of a detergent,
b) mechanically treating said suspension to obtain particles comprising the active ingredient with an effective average size of less than about 5000 nm,
c) contacting said active ingredient or suspension with a first polyelectrolyte or with a polyelectrolyte complex during and/or before mechanically treating,
d) optionally contacting said suspension with a one or more second or further polyelectrolytes during, before and/or after mechanically treating, and
e) optionally drying said suspension.

### Detailed description of the invention

Subject of the present invention is a method for the production of a nanoparticulate pharmaceutical composition comprising an active ingredient comprising the steps of:
a) suspending in a liquid dispersion medium a poorly soluble active ingredient without the presence of a detergent,
b) mechanically treating said suspension to obtain particles comprising the active ingredient with an effective average size of less than about 5000 nm, preferably less than about 4000 nm, more preferably less than about 3000 nm, even more preferably less than about 1000 nm and most preferably less than about 800 nm,
c) contacting said active ingredient or suspension with a first polyelectrolyte or with a polyelectrolyte complex during and/or before mechanically treating,
d) optionally contacting said suspension with a one or more second or further polyelectrolytes during, before and/or after mechanically treating, and
e) optionally drying said suspension.

One particularly preferred embodiment the invention relates to a method for the production of a nanoparticulate pharmaceutical composition comprising an active ingredient comprising the steps of:
a) suspending in a liquid dispersion medium a poorly soluble active ingredient without the presence of a detergent,
b) mechanically treating said suspension to obtain particles comprising the active ingredient with an effective average size of less than about 5000 nm, preferably less than about 4000 nm, more preferably less than about 3000 nm, even more preferably less than about 1000 nm and most preferably less than about 800 nm,
c) contacting said active ingredient or suspension with a first polyelectrolyte during and/or before mechanically treating,
d) optionally contacting said suspension with a one or more second or further polyelectrolytes during, before and/or after mechanically treating, and
e) optionally drying said suspension.

In a preferred embodiment of the invention the step of contacting said suspension with a one or more second or further polyelectrolytes during, before and/or after mechanically treating is mandatory. Thus, this preferred embodiment relates to a method for the production of a nanoparticulate pharmaceutical composition comprising an active ingredient comprising the steps of:
a) suspending in a liquid dispersion medium a poorly soluble active ingredient without the presence of a detergent,
b) mechanically treating said suspension to obtain particles comprising the active ingredient with an effective average size of less than about 5000 nm,
c) contacting said active ingredient or suspension with a first polyelectrolyte or with a polyelectrolyte complex during and/or before mechanically treating, and
d) contacting said suspension with a one or more second or further polyelectrolytes during, before and/or after mechanically treating.

The poorly soluble active ingredient has preferably a solubility in the liquid dispersion medium, e.g. in water of less than 10 g/L, preferably 1 g/L, more preferably less than about 250 mg/L, most preferably less than 100 mg/L at processing temperature, e.g. room temperature. The preferred liquid dispersion medium is water; however other liquid media in which the active ingredient is poorly soluble and dispersible including aqueous salt solutions or aqueous mixtures of solvents such as ethanol, benzyl alcohol, dimethyl sulfoxide, chlorobutanol, glycerin, thioglycerol and polyethylene glycol. The pH of the aqueous dispersion media can be adjusted by techniques known in the art.

A detergent according to the present invention is defined as an amphiphilic non-polyionic compound that reduces surface tension of materials.

The average size of the particles is preferably less than about 5000 nm, more preferably less than about 3000 nm, even more preferably less than about 1000 nm most preferably less than about 800 nm.

The average size of the particles is measured by the following procedure: Photon Correlation Spectroscopy (PCS) for expected average sizes below 3 µm, or by Laser Diffractometry (LD) for expected larger particle sizes.

A polyelectrolyte according to the present invention is a polymer whose repeating units bear an electrolyte group. These groups will dissociate in aqueous solutions, making the polymers charged.

A typical wet mill configuration involves slurry circulated through a high shear mixer (ULTRA-TURRAX® and Mills from IKA® Werke GmbH&Co.KG), or using beads or basket mills (DISPERMAT® and TOROUSMILL® from VMA-Getzmann GmbH), or planetary micro mills (PULVERISETTE 7, 6, 5, 4, 0, Classic Line/Premium Line from Fritsch GmbH) at rotation speeds of up to 1100 rpm. In preferred embodiments 2.5 mm zirconium oxide milling beads, 2.0 mm zirconium oxide milling beads, 1.8 mm zirconium oxide milling beads, 1.5 mm zirconium oxide milling beads and/or 0.5 mm zirconium oxide milling beads are used (diameters of the beads). Preferably, milling beads with a diameter of from about 1.5 mm to about 1.8 mm are used. During milling, the beads may be exchanged, for instance when adding the further (e.g. second) polyelectrolyte the beads are changed from 2.5 to 0.5 mm zirconium beads. In another preferred embodiment, the milling beads are not exchanged during the milling process. Milling times may be varied. Preferred milling times are for example 10, 20, 30, 40, 50 or 60 minutes independently for each milling step.

It is preferred in the method of the present invention that the suspension is contacted with the first polyelectrolyte before or during the mechanical treatment, and the suspension is contacted with the second or further polyelectrolyte during the mechanical treatment. It is preferred that when contacting the suspension with the first and/or second or further polyelectrolyte, respectively, said polyelectrolyte is dissolved in an aqueous solution, i.e. an aqueous solution of the polyelectrolyte is added to the suspension. In another preferred embodiment, the first polyelectrolyte is added before milling as an aqueous solution directly to the active ingredient which is in powder form, thereby suspending said poorly soluble active ingredient. Preferably, the second, oppositely charged, polyelectrolyte is then added during milling to the suspension.

It is preferred that the first and further (e.g. second) polyelectrolyte form polyelectrolyte complexes during the method of the invention and the pharmaceutical compositions of the invention thus preferably comprise polyelectrolyte complexes.

Preferably the method according to the present invention is carried out without any intermediate washing steps. In a preferred embodiment of the method for the production of a nanoparticulate pharmaceutical composition according to the present invention, the method comprises a dilution step at the end of the process to obtain the formed slurry.

In a preferred method according to the present invention, step a) suspending a poorly soluble active ingredient is carried out without the presence of a detergent.

In a preferred embodiment of the method according to the invention the method is carried out without a detergent selected from the group comprising soaps, fatty acid salts, Sodium dodecyl sulfate (SDS), ammonium lauryl sulfate, and other alkyl sulfate salts, Sodium lauryl sulfate, Sodium laureth sulfate (also known as sodium lauryl ether sulfate (SLES)), Alkyl benzene sulfonate, Cetyl trimethylammonium bromide (CTAB) a.k.a. hexadecyl trimethyl ammonium bromide, and other alkyltrimethyl ammonium salts, Cetylpyridinium chloride (CPC), Polyethoxylated tallow amine (POEA), Benzethonium chloride (BZT), Dodecyl betaine, Dodecyl dimethylamine oxide, Cocamidopropyl betaine, Coco ampho glycinate, Alkyl polyglucosides, including: Octyl glucoside, Decyl maltoside and Cocamide MEA, cocamide DEA.

In another preferred embodiment of the method according to the invention mechanically treating is selected from the group comprising wet milling, high-shear mixing and high-pressure homogenization.

Most preferred is wet milling as mechanically treating. According to the invention several improvements may be applied to the milling process as outlined in the following.
The hydrodynamics during milling can be influenced by the addition of polyelectrolytes, improving the yield of milling and thus the control of the particle size. The polyelectrolytes may serve as additional nanoparticulate abrasive agents during milling. The presence of polyelectrolytes during the milling process may lead to transitional coating of the grist, the walls of the mill and the grinding balls, respectively, thereby improving the grinding process and the milling results. The polyelectrolytes also serve as lubricants during milling resulting in a reduction of abrasion of the milling equipment and a decrease in contamination of the grist.

In a preferred embodiment of the method according to the invention the first polyelectrolyte is selected from the group comprising water-soluble cationic or anionic polysaccharides, poly-amino acids, peptides, proteins, nucleic acids and corresponding salts thereof. Most preferred first polyelectrolytes are selected from the group comprising xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits (e.g. Eudragit S, Eudragit E), protamine, albumins (e.g. human or bovine serum albumin (HSA or BSA)), casein, gelatine (e.g. gelatine A, gelatine B), collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized (for example etherified, esterified) derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

In a preferred embodiment of the method according to the invention the second or further polyelectrolyte is selected from the group comprising water-soluble cationic or anionic polysaccharides, poly-amino acids, peptides, proteins, nucleic acids and corresponding salts thereof. Most preferred second or further polyelectrolytes are selected from the group comprising xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits (e.g. Eudragit S, Eudragit E), protamine, albumins (e.g. HSA or BSA), casein, gelatine (e.g. gelatine A, gelatine B), collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized (for example etherified, esterified) derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.. Most preferably the second polyelectrolyte is oppositely charged to the first polyelectrolyte, i.e. when the first polyelectrolyte is a polyanion, the second polyelectrolyte is a polycation and vice versa. When two or more further polyelectrolytes are used, it is preferred that the first and third (and if applicable the other odd-numbered polyelectrolytes) polyelectrolyte have the same charge and the second (and if applicable the other even-numbered polyelectrolytes) have opposite charges as compared to the first polyelectrolyte. In one preferred embodiment, the suspension is contacted with a second polyelectrolyte and no further polyelectrolytes. Most preferably the second polyelectrolyte is oppositely charged than the first polyelectrolyte.

In one preferred embodiment, the first polyelectrolyte is Eudragit E and the second polyelectrolyte is Eudragit S. In another preferred embodiment the first polyelectrolyte is Eudragit S and the second polyelectrolyte is Eudragit E. In yet another preferred embodiment, the first polyelectrolyte is protamine and the second polyelectrolyte is carboxymethyl cellulose. In yet another preferred embodiment, the first polyelectrolyte is protamine sulfate and the second polyelectrolyte is chondroitin sulfate.

In another preferred embodiment of the method according to the invention, drying comprises a method selected from the group comprising freeze-drying, spray-drying, evaporation, heating, vacuum-drying or combinations thereof. In a preferred embodiment of the invention, the step of drying is mandatory. Preferably, the methods of the invention comprise a step of drying said suspension.

In another preferred embodiment of the method according to the invention a solubilizer is present in said suspension at a concentration below about 2%, most preferred below about 1%. Preferably the solubilizer is selected from a group comprising polyvinyl pyrrolidone, polyethylene glycol, polypropylen glycol, polyethylene glycol 660 hydroxystearate, polysorbat, benzyl alcohol, ethanol, polyvinyl alcohol, Lipoid, ethyl oleate, transcutol, glycofurol, miglyol.

In another preferred embodiment of the method according to the invention the poorly soluble active ingredient is a poorly soluble drug according to groups II or IV of the Biopharmaceutics Classification System (BCS) (FDA).
The active ingredient may for example be selected from the group comprising
- Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCl, Qinapril, Ramipril, Simvastatin, Telmisartan, Trandolapril, Valsartan and other cardio-vascular active drugs;
- Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir and other anti-infective drugs including anti-bacterial, anti-viral, anti fungal and anti-parasitic drugs;
- Cisplatin, Carboplatin, Docetaxel, Etoposide, Exemestane, Idarubicin, Irinotecan, Melphalan, Mercaptopurine, Mitotane, Paclitaxel, Valrubicin, Vincristine and other drugs used in oncology;
- Azathioprine, Tacrolimus, Cyclosporine, Pimecrolimus, Sirolimus and other immonosupressive drugs;
- Clozapine, Entacapone, Fluphenazine, Imipramine, Nefazodone, Olanzapine, Paroxetine, Pimozide, Sertraline, Triazolam, Zaleplon, Ziprasidoneand, Risperidone, Carbamazepine and other drugs for CNS indications;
- Danazol, Dutasteride, Medroxyprogesterone, Estradiol, Raloxifene, Sildenafil, Tadalafil, Testosterone, Vardenafil and other drugs used for reproductive health;
- Celecoxib, Dihydroergotamine Mesylate, Eletriptan, Ergoloidmesylates, Ergotamine-tartrate, Nabumetone, Ibuprofen, Ketoprofen, Triamcinolone, Triamcinolone acetonide and other anti-inflammatory and analgesic drugs;
- Bosentan, Budesonide, Desloratadine, Fexofenadin, Fluticasone, Loratadine, Mometasone, Salmeterol Xinafoate, Triamcinolon Acetonide, Zafirlukast and other drugs for respiratory indications; and
- Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide, Trizanidine hydrochloride and other drugs for various indications including in particular gastro-intestinal disorders, diabetes and dermatology indications.

In another preferred embodiment of the method according to the invention the concentration of the poorly soluble active ingredient in the liquid medium is higher than about 0.1%, preferably higher than about 1%, even more preferably higher than about 5%, most preferably higher than about 10% up to about 65% (w/w).

According to the invention several improvements may be applied to the process for the production of a nanoparticulate pharmaceutical composition according to the present invention.
The presence of polyelectrolytes during production of the particles leads to an increase in stability of the suspension against Ostwald ripening.
The mean distance between individual particles of active agent is increased by the presence of polyelectrolyte complexes adsorbed to the drug surface and in the dispersion medium, thereby increasing their surface stability and preventing aggregation. Also the stability of the suspension is increased during sterilisation procedures (e.g. autoclaving, gamma radiation treatment) and drying procedures (e.g. freeze-drying, spray-drying, vacuum drying and heat drying) when polyelectrolytes are present.
In addition, rheological properties of dried powder of the nanoparticulate pharmaceutical composition are enhanced by the presence of polyelectrolytes, thereby enhancing the volumetric dosing, the further galenic processing into solid dosage forms (e.g. filling into capsules, compression into tablets, and incorporation into a solid matrix). Likewise, the incorporation of the nanoparticulate pharmaceutical composition of the present invention into the hydrophilic phase of a heterogeneous system (e.g. emulsion, hydrogel, cream) is enhanced by the presence of polyelectrolytes.

Pharmaceutical composition obtainable according to the method of the invention comprising
a. a poorly soluble active ingredient,
b. a first polyelectrolyte, and
c. one or more second or further polyelectrolytes,
wherein the pharmaceutical composition does not comprise a detergent and wherein the pharmaceutical composition is in a nanoparticulate form with effective average particle sizes of less than about 5000 mm, preferably less than about 4000 nm, more preferably less than about 3000 nm, even more preferably less than about 1000 nm and most preferably less than about 800 nm, and the active ingredient forms the core of the particle and wherein the first and optionally further polyelectrolytes are arranged in alternating layers of polyelectrolytes with opposite charges around the active ingredient and structures of polyelectrolyte complexes are formed on the surface of said pharmaceutical composition.

Polyelectrolyte complexes are amorphous salts between oppositely charged polyelectrolytes. These polyelectrolyte complexes may be formed on the surface of said pharmaceutical composition.

In a preferred embodiment of the pharmaceutical composition the first electrolyte is selected from the group comprising water-soluble cationic or anionic polysaccharides, poly-amino acids, peptides, proteins, nucleic acids and corresponding salts thereof. Most preferred first polyelectrolytes are selected from the group comprising xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits (e.g. Eudragit S, Eudragit E), protamine, albumins, casein, gelatine (e.g. gelatine A, gelatine B), collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized (for example etherified, esterified) derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

In a preferred embodiment of the pharmaceutical composition the second or further polyelectrolyte is selected from the group comprising water-soluble cationic or anionic polysaccharides, poly-amino acids, peptides, proteins, nucleic acids and corresponding salts thereof. Most preferred first polyelectrolytes are selected from the group comprising xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits (e.g. Eudragit S, Eudragit E), protamine, albumins, casein, gelatine (e.g. gelatine A, gelatine B), collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized (for example etherified, esterified) derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

Negatively charged polyelectrolytes may for example be selected from the group comprising xylan polysulfate, dextran sulfate, poly(amino acids) such as polyaspartic acid or polyglutamic acid, polysaccharide polysulfate such as sulfate of starch hydrolysate, inulin, hydroxyethylstarch, polysaccharide polysulfonate, polysaccharide polyphosphate, carboxymethylcellulose, gelatin B, collagen, Eudragit S and polyphosphates.

Positively charged polyelectrolytes may for example be selected from the group comprising poly-L-lysine, poly-α, β-(2-dimethylaminoethyl)-D,L-aspartamide, chitosan, lysine octadecyl ester, aminated dextran, aminated cyclodextrin, aminated cellulose ether, protamine (sulfate), gelatin A, Eudragit E, casein, nucleic acid (e.g. DNA, RNA, LNA or PNA) and aminated pectin.

Preferred polyelectrolyte complexes may for instance be selected from the group comprising protamine/carboxymethyl cellulose, protamine/gelatine B, protamine/chondroitin sulphate, protamine/HAS, protamine/alginate, protamine/carragenate, protamine/Eudragit S, HSA/gelatine, Eudragit E/Eudragit S, Eudragit E/carboxymethyl cellulose, Eudragit E/chondroitin sulphate, Eudragit E/carragenate, Eudragit E/alginate, chitosan/carboxymethyl cellulose, chitosan/chondroitin sulfate, chitosan/alginate, chitosan/carragenate, chitosan/gelatine.

In a preferred embodiment of the pharmaceutical composition the poorly soluble active ingredient is a poorly soluble drug according to groups II or IV of the Biopharmaceutics Classification System (BCS) (FDA). The active ingredient may for example be selected from the group comprising:
- Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCl, Qinapril, Ramipril, Simvastatin, Telmisartan, Trandolapril, Valsartan and other cardio-vascular active drugs;
- Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir and other anti-infective drugs including anti-bacterial, anti-viral, antifungal and anti-parasitic drugs;
- Cisplatin, Carboplatin, Docetaxel, Etoposide, Exemestane, Idarubicin, Irinotecan, Melphalan, Mercaptopurine, Mitotane, Paclitaxel, Valrubicin, Vincristine and other drugs used in oncology;
- Azathioprine, Tacrolimus, Cyclosporine, Pimecrolimus, Sirolimus and other immonosupressive drugs;
- Clozapine, Entacapone, Fluphenazine, Imipramine, Nefazodone, Olanzapine, Paroxetine, Pimozide, Sertraline, Triazolam, Zaleplon, Ziprasidoneand, Risperidone, Carbamazepine and other drugs for CNS indications;
- Danazol, Dutasteride, Medroxyprogesterone, Estradiol, Raloxifene, Sildenafil, Tadalafil, Testosterone, Vardenafil and other drugs used for reproductive health;
- Celecoxib, Dihydroergotamine Mesylate, Eletriptan, Ergoloidmesylates, Ergotamine-tartrate, Nabumetone, Ibuprofen, Ketoprofen, Triamcinolone, Triamcinolone acetonide and other anti-inflammatory and analgesic drugs;
- Bosentan, Budesonide, Desloratadine, Fexofenadin, Fluticasone, Loratadine, Mometasone, Salmeterol Xinafoate, Triamcinolon Acetonide, Zafirlukast and other drugs for respiratory indications; and
- Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide, Trizanidine hydrochloride and other drugs for various indications including in particular gastro-intestinal disorders, diabetes and dermatology indications.

In a preferred embodiment of the pharmaceutical composition the content of the poorly soluble active ingredient is lower than 65% for fluid systems or lower than 98% for dried systems.
Advantages of the pharmaceutical compositions of the present invention include the increased stability of suspension dosage forms as compared to pharmaceutical compositions that do not comprise polyelectrolytes. The choice of the polyelectrolytes used allows for the distinction of the pharmaceutical compositions of the present invention from other compositions (e.g. counterfeit pharmaceuticals), e.g. by comparing structural features like surface roughness, e.g. by analysis of scanning electron microscopy (SEM) images.
Also possible is the functionalization of the drug particles by the properties of the polyelectrolytes chosen, e.g. for targeting, mediation of mucoadhesion and permeation enhancement. In a preferred embodiment the outer most polyelectrolyte (i.e. the polyelectrolyte added at last) is selected from the group of chondroitin sulfate, carrageenan, alginate, carboxymethylcellulose and chitosan. This serves the mediation of mucoadhesion and permeation enhancement.

The incorporation of polyelectrolytes also allows for the functionalization of the surface of the drug particles with charged groups in order to allow for the iontophoretic application of the pharmaceutical compositions of the present invention.

Further, the polyelectrolyte complexes comprised in the pharmaceutical composition may act as hygroscopic agents to bind moisture, thereby protecting potentially sensitive active agents e.g. from hydrolysis. Polyelectrolytes comprised in the pharmaceutical compositions of the present invention may also have biocide properties, e.g. PSS, Carrageenan and Chitosan have antibiotic properties.

The pharmaceutical compositions according to the present invention may for example be prepared for intravenous, intramuscular, subcutaneous, intracardial, intrathecal, intracranial, intravesical, intrabursal, intraocular, intravitreal and intra-articular injection or implant; intravenous infusion; oral, buccal, sublingual, periodontal, vaginal, intrauterine, rectal, pulmonary, nasal, inhalation, intraocular, ophthalmic, auricular, transdermal and topical application. Preferred applications of the pharmaceutical compositions of the invention are oral, subcutaneous, intramuscular, intravenous and iontophoretic applications.

The pharmaceutical compositions of the present invention may be used as medicaments. The present invention relates in another particular aspect to the pharmaceutical composition according to the invention for the use as a medicament, wherein the composition is applied orally, subcutaneously, intramuscularly or intravenously. The present invention relates in another particular aspect to the pharmaceutical composition according to the invention for the use as a medicament, wherein the composition is applied or delivered iontophoretically. Thus, the pharmaceutical compositions of the present invention may in a particular embodiment be applied iontophoretically.

Normally, only charged active ingredients can be delivered via iontophoresis. Poorly soluble compounds are neutral unless treated with the methods of this invention, whereby the added polyelectrolytes may confer a charge at the surface of the nanoparticular pharmaceutical compositions and thus such nanoparticular pharmaceutical compositions may be delivered via iontophoresis.

### Examples

### Example 1

The purpose of this example was to prepare a nanoparticular suspension of candesartan cilexetil exclusively stabilized by oppositely charged methacrylate polyelectrolytes. Concretely, Eudragit E, a cationic polymer with dimethylaminoethyl methacrylate as a functional group, and Eudragit S, an anionic polymer with methacrylic acid as a functional group, were incorporated to the drug suspension during the mechanical treatment for size reduction. Candesartan cilexetil is a selective AT₁ subtype angiotensin II receptor antagonist, practically insoluble in water, indicated for the treatment of hypertension.
A slightly acidic aqueous solution of 1% Eudragit E was prepared in water with a pH of 4. An alkaline aqueous solution of 1% Eudragit S was prepared in water with a pH of 8. 150 mg of candesartan cilexetil were mixed with 3 ml of the 1% Eudragit E solution in the 20 ml chamber of a planetary mill (Pulverisette-7, manufactured by Fritsch GmbH) and milled with 2.5 mm zirconium oxide beads for 20 min at 800 rpm. Then the beads were changed by 0.5 mm zirconium oxide beads and milling was continued for additional 20 min at 800 rpm while 3 ml of the 1% Eudragit S solution were incorporated into the system. The resulting dispersion was stable when was added to 0.1N HCl or to phosphate buffer solution pH 7.4, and when stored at room temperature. The average particle size measured by photon correlation spectroscopy was 275.5 nm and the particles displayed a negative zeta potential of -28.1 mV.

### Example 2

Example 1 was repeated except that the order of addition of the polyelectrolyte solutions was inverted. Namely, the Eudragit S solution was added at the initial phase of the milling process and the Eudragit E solution was added after changing the milling beads. The resulting dispersion was stable when was added to 0.1N HCl or to phosphate buffer solution pH 7.4, and when stored at room temperature. The average particle size measured by photon correlation spectroscopy was 334.8 nm and the particles displayed a positive zeta potential of +1.2 mV.

### Example 3

Example 1 was repeated except that the overall solid content in the system was increased. 3750 mg of candesartan cilexetil were mixed with 7.5 ml of a 5% Eudragit E solution at pH 4 in the 45 ml chamber of a planetary mill (Pulverisette-7, manufactured by Fritsch GmbH) and milled with 1.5 mm zirconium oxide beads for 20 min at 800 rpm. Then 7.5 ml of a 5% Eudragit S solution at pH 8 were incorporated into the system and the milling was continued for 40 min at 800 rpm. The resulting dispersion was stable when was added to 0.1N HCl or to phosphate buffer solution pH 7.4, and when stored at room temperature. The average particle size measured by photon correlation spectroscopy was 597 nm.

### Example 4

The purpose of this example was to prepare a nanoparticular suspension of candesartan cilexetil exclusively stabilized by oppositely charged natural occurring polyelectrolytes. Concretely, protamine a cationic arginine-rich peptide and carboxymethyl cellulose an anionic polyelectrolyte were incorporated to the drug suspension during the mechanical treatment for size reduction.
1670 mg of candesartan cilexetil were mixed with 2 ml of a 0.67% protamine sulfate aqueous solution in the 20 ml chamber of a planetary mill (Pulverisette-7, manufactured by Fritsch GmbH) and milled with 2.5 mm zirconium oxide beads for 20 min at 800 rpm. Then 2 ml of a 3.3% carboxymethyl cellulose aqueous solution were incorporated into the system and milling was continued for additional 20 min or additional 40 min at 800 rpm. The average particle size of the dispersion was improved with longer milling times after the addition of the second polyelectrolyte due to the formation of stabilizing polyelectrolyte complexes which enhanced the milling performance. The average particle size of the dispersion measured by photon correlation spectroscopy was 1073 nm after 20 min milling time upon addition of the second polyelectrolyte and 582 nm after 40 min milling time upon the addition of the second polyelectrolyte. The resulting dispersion was stable when stored at room temperature, when dried via spray-drying and when added to a 0.1N HCl solution or to phosphate buffer solution pH 7.4. The particles in dispersion displayed a negative zeta potential of -42.7 mV.

### Example 5

Example 4 was repeated except that the polyanionic material employed was chondroitin sulphate. 1670 mg of candesartan cilexetil were mixed with 2 ml of a 0.67% protamine sulfate aqueous solution in the 20 ml chamber of a planetary mill (Pulverisette-7, manufactured by Fritsch GmbH) and milled with 2.5 mm zirconium oxide beads for 20 min at 800 rpm. Then 2 ml of a 3.3% chondroitin sulphate aqueous solution were incorporated into the system and milling was continued for additional 40 min at 800 rpm. The average particle size of the dispersion was improved after the addition of the second polyelectrolyte due to the formation of stabilizing polyelectrolyte complexes which enhanced the milling performance. The average particle size of the dispersion was 903 nm and the zeta potential was -44.5 mV.

### Example 6

Different nanoparticulate suspensions of candesartan cilexetil are formulated with Eudragit E and Eudragit S, or with protamine and carboxymethyl cellulose, or with protamine and chondroitin sulphate, analogue as stated in the previous examples. These formulations are administered orally to Wistar male rats and compared to the performance of a control candesartan cilexetil formulation. The pharmacokinetic evaluation is carried out upon oral administration in solid and in fluid form.

### Example 7

Different nanoparticulate suspensions of candesartan cilexetil are formulated with protamine and carboxymethyl cellulose, or with protamine and chondroitin sulphate, analogue as stated in the previous examples. A nanosuspension formulation is administered intravenously to Wistar male rats. The pharmacokinetic and biodistribution evaluation upon the intraveneous administration of the polyelectrolyte complex-stabilized nanosuspension of candesartan cilexetil is carried out and compared to the performance of a control solution of candesartan cilexetil with state-of-the-art excipients.

## Claims

1. Method for the production of a nanoparticulate pharmaceutical composition comprising an active ingredient comprising the steps of:
a) suspending in a liquid dispersion medium a poorly soluble active ingredient without the presence of a detergent,
b) mechanically treating said suspension to obtain particles comprising the active ingredient with an effective average size of less than about 5000 nm,
c) contacting said suspension with a first polyelectrolyte or with a polyelectrolyte complex during and/or before mechanically treating, and
d) optionally contacting said suspension with one or more second or further polyelectrolytes during, before and/or after mechanically treating.

2. Method according to claim 1, wherein said suspension is contacted with one or more second or further polyelectrolytes during, before and/or after mechanically treating and the second polyelectrolyte is oppositely charged to the first polyelectrolyte.

3. Method according to claim 1 or 2, wherein the step of contacting said suspension with one or more second or further polyelectrolytes during, before and/or after mechanically treating is mandatory.

4. Method according to claims 1 to 3, wherein the liquid dispersion medium is selected from the group comprising water, aqueous salt solutions and aqueous mixtures of solvents such as ethanol, benzyl alcohol, dimethyl sulfoxide, chlorobutanol, glycerin, thioglycerol and polyethylene glycol.

5. Method according to claims 1 to 4, wherein the method additionally comprises the step of drying said suspension.

6. Method according to claims 1 to 5, wherein said poorly soluble active ingredient has a solubility in water of less than 10 g/L.

7. Method according to claims 1 to 6, wherein the method is carried out without the presence of a detergent selected from the group comprising soaps, fatty acid salts, Sodium dodecyl sulfate (SDS), ammonium lauryl sulfate, and other alkyl sulfate salts, Sodium laureth sulfate (also known as sodium lauryl ether sulfate (SLES)), Alkyl benzene sulfonate, Cetyl trimethylammonium bromide (CTAB) a.k.a. hexadecyl trimethyl ammonium bromide, and other alkyltrimethylammonium salts, Cetylpyridinium chloride (CPC), Polyethoxylated tallow amine (POEA), Benzethonium chloride (BZT), Dodecyl betaine, Dodecyl dimethylamine oxide, Cocamidopropyl betaine, Coco ampho glycinate, Alkyl polyglucosides, including Octyl glucoside, Decyl maltoside, Cocamide MEA, and cocamide DEA.

8. Method according to claims 1 to 7, wherein mechanically treating is selected from the group comprising wet milling, high-shear mixing and high-pressure homogenization.

9. Method according to claims 1 to 8, wherein the first electrolyte is selected from the group comprising water-soluble cationic or anionic polysaccharides, peptides, proteins, nucleic acids and corresponding salts thereof, xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits, protamine, albumins, casein, gelatine, collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

10. Method according to claims 1 to 9, wherein the second or further polyelectrolyte is selected from the group comprising water-soluble cationic or anionic polysaccharides, peptides, proteins, nucleic acids and corresponding salts thereof, xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits, protamine, albumins, casein, gelatine, collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

11. Method according to claims 1 to 10, wherein a solubilizer selected from the group consisting of polyvinyl pyrrolidone, polyethylene glycol, polypropylen glycol, polyethylene glycol 660 hydroxystearate, polysorbat, benzyl alcohol, ethanol, polyvinyl alcohol, Lipoid, ethyl oleate, transcutol, glycofurol, miglyol is present in said suspension.

12. Method according to claims 1 to 11, wherein the poorly soluble active ingredient is selected from a group comprising
- Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCl, Qinapril, Ramipril, Simvastatin, Telmisartan, Trandolapril, Valsartan and other cardio-vascular active drugs;
- Cisplatin, Carboplatin, Paclitaxel, Docetaxel, Vincristine, Etoposide and other antineoplastic compounds used to treat cancer.
- Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir and other anti-infective drugs including anti-bacterial, anti fungal and anti-parasitic drugs;
- Cisplatin, Docetaxel, Etoposide, Exemestane, Idarubicin, Irinotecan, Melphalan, Mercaptopurine, Mitotane, Paclitaxel, Valrubicin and other drugs used in oncology;
- Azathioprine, Tacrolimus, Cyclosporine, Pimecrolimus, Sirolimus and other immonosupressive drugs;
- Clozapine, Entacapone, Fluphenazine, Imipramine, Nefazodone, Olanzapine, Paroxetine, Pimozide, Sertraline, Triazolam, Zaleplon, Ziprasidoneand, Risperidone, Carbamazepine and other drugs for CNS indications;
- Danazol, Dutasteride, Medroxyprogesterone, Estradiol, Raloxifene, Sildenafil, Tadalafil, Testosterone, Vardenafil and other drugs used for reproductive health;
- Celecoxib, Dihydroergotamine Mesylate, Eletriptan, Ergoloidmesylates, Ergotamine-tartrate, Nabumetone, Ibuprofen, Ketoprofen, Triamcinolone, Triamcinolone acetonide and other anti-inflammatory and analgesic drugs;
- Bosentan, Budesonide, Desloratadine, Fexofenadin, Fluticasone, Loratadine, Mometasone, Salmeterol Xinafoate, Triamcinolon Acetonide, Zafirlukast and other drugs for respiratory indications; and
- Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide, Trizanidine hydrochloride and other drugs for various indications including in particular gastro-intestinal disorders, diabetes and dermatology indications.

13. Method according to claims 1 to 12, wherein the concentration of the poorly soluble active ingredient in the liquid medium is higher than about 0.1 % (w/w).

14. Pharmaceutical composition obtainable according to the method according to any one of the claims 1 to 13 comprising
a. a poorly soluble active ingredient,
b. a first polyelectrolyte, and
c. one or more second or further polyelectrolytes,
wherein the pharmaceutical composition does not comprise a detergent and wherein the pharmaceutical composition is in a nanoparticulate form with effective average particle sizes of less than about 5000 mm and the active ingredient forms the core of the particle and wherein the first and optionally further polyelectrolytes are arranged in alternating layers of polyelectrolytes with opposite charges around the active ingredient and structures of polyelectrolyte complexes are formed on the surface of said pharmaceutical composition.

15. Pharmaceutical composition according to claim 14, wherein the first electrolyte is selected from the group comprising water-soluble cationic or anionic polysaccharides, peptides, proteins, nucleic acids and corresponding salts thereof, xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits, protamine, albumins, casein, gelatine, collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-α,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

16. Pharmaceutical composition according to claims 14 or 15, wherein the second or further polyelectrolyte is selected from the group comprising water-soluble cationic or anionic polysaccharides, peptides, proteins, nucleic acids and corresponding salts thereof, xylan polysulfates, dextran sulfates, poly(amino acids) such as polyaspartic acid, poly-arginine, poly-lysine or polyglutamic acid, polysaccharide polysulfates such as sulfates of starch hydrolysates, inulin, hydroxyethylstarches, polysaccharide polysulfonates, polysaccharide polyphosphates, polyphosphates, Eudragits, protamine, albumins, casein, gelatine, collagen, oligonucleotides, polymethacrylic acid, polyacrylic acid, chitosan, pectin, carboxymethylcellulose, alginate, carrageenan, hyaluronic acid, chondroitin sulfate, dextrane sulphate, heparine, poly-β,β-(2-dimethylaminoethyl)-D,L-aspartamide, chitosan, lysine octadecyl ester, aminated dextrans, aminated cyclodextrins, aminated cellulose ethers, aminated pectins, polystyrenesulfonate and corresponding salts thereof and in each case partially hydrophobized derivatives of xylan polysulfate, polysulfates of other polysaccharides such as, for example, starch hydrolysates, inulin, hydroxyethylstarches, dextrans; of poly(amino acids) such as polyaspartic acid or polyglutamic acid, and of polysaccharide polysulfonates, polysaccharide polyphosphonates, polyphosphates.

17. Pharmaceutical composition according to claims 14 to 16, wherein the poorly soluble active ingredient is selected from a group comprising
- Atorvastatin, Amiodarone, Candesartan-Cilexetil, Carvedilol, Clopidogrel bisulfate, Dipyridamole, Eprosartan mesylate, Epierenone, Ezetimibe, Felodipine, Furosemide, Isradipine, Lovastatin, Metolazone, Nicardipine, Nisoldipine Olmesartan medoxomil, Propafenone HCl, Qinapril, Ramipril, Simvastatin, Telmisartan, Trandolapril, Valsartan and other cardio-vascular active drugs;
- Cisplatin, Carboplatin, Paclitaxel, Docetaxel, Vincristine, Etoposide and other antineoplastic compounds used to treat cancer.
- Acyclovir, Adefovir, Dipivoxil, Amphotericin, Amprenavir, Cefixime, Ceftazidime, Clarithromycin, Clotrimazole, Efavirenz, Ganciclovir, Itraconazole, Norfloxacin, Nystatin Ritonavir, Saquinavir and other anti-infective drugs including anti-bacterial, anti fungal and anti-parasitic drugs;
- Cisplatin, Docetaxel, Etoposide, Exemestane, Idarubicin, Irinotecan, Melphalan, Mercaptopurine, Mitotane, Paclitaxel, Valrubicin and other drugs used in oncology;
- Azathioprine, Tacrolimus, Cyclosporine, Pimecrolimus, Sirolimus and other immonosupressive drugs;
- Clozapine, Entacapone, Fluphenazine, Imipramine, Nefazodone, Olanzapine, Paroxetine, Pimozide, Sertraline, Triazolam, Zaleplon, Ziprasidoneand, Risperidone, Carbamazepine and other drugs for CNS indications;
- Danazol, Dutasteride, Medroxyprogesterone, Estradiol, Raloxifene, Sildenafil, Tadalafil, Testosterone, Vardenafil and other drugs used for reproductive health;
- Celecoxib, Dihydroergotamine Mesylate, Eletriptan, Ergoloidmesylates, Ergotamine-tartrate, Nabumetone, Ibuprofen, Ketoprofen, Triamcinolone, Triamcinolone acetonide and other anti-inflammatory and analgesic drugs;
- Bosentan, Budesonide, Desloratadine, Fexofenadin, Fluticasone, Loratadine, Mometasone, Salmeterol Xinafoate, Triamcinolon Acetonide, Zafirlukast and other drugs for respiratory indications; and
- Dronabinol, Famotidine, Glyburide, Hyoscyamine, Isotretinoin, Megestrol, Mesalamine, Modafinil, Mosapride, Nimodipine, Perphenazine, Propofol, Sucralfate, Thalidomide, Trizanidine hydrochloride and other drugs for various indications including in particular gastro-intestinal disorders, diabetes and dermatology indications.

18. Pharmaceutical composition according to claims 14 to 17, wherein the content of the poorly soluble active ingredient is higher than about 0.1 % (w/w).

19. Pharmaceutical composition according to any one of the claims 14 to 18 for the use as a medicament, wherein the composition is applied or delivered iontophoretically.
